# DEMANDE DE BREVET EUROPEEN

(11) **EP 1 312 650 A2**
(43) Date de publication de la demande: **21.05.2003**
(21) Numéro de dépôt: 02292784.2
(22) Date de dépôt: 07.11.2002
(51) Int. Cl.: C08L 83/08, A61K 7/09

(54) **Procédé de déformation permanente des cheveux mettant en oeuvre des silicones aminées particulières**

(30) Priorité: 08.11.2001 FR 0114479
(71) Demandeur: L'OREAL, 75008 Paris (FR)
(72) Inventeur: Devin-Baudoin, Priscille, 92170 Vanves (FR); Sabbagh, Anne, 92500 Rueil Malmaison (FR)
(74) Mandataire: Bourdeau, Françoise

(57) **Abrégé**

L'invention concerne un procédé de déformation permanente des fibres kératiniques comprenant l'application sur les fibres kératiniques, avant l'opération de réduction et/ou après l'opération de fixation, d'une composition cosmétique de pré-traitement et/ou de post-traitement comprenant, dans un véhicule cosmétiquement acceptable, au moins une silicone aminée aminoéthyliminoalkyl (C₄-C₈).

## Description

La présente invention concerne un procédé déformation permanente des fibres kératiniques, en particulier des cheveux, ledit procédé étant notamment utilisable dans les salons de coiffure, professionnels ou par des particuliers, via la commercialisation de kits.

Au sens de la présente invention, on entend par *« procédé de déformation permanente »*, tout procédé durable dans le temps, de mise en forme des cheveux, de frisage, de défrisage ou de décrêpage.

On entend par *« fibres kératiniques »*, en particulier, les cheveux, les cils, les sourcils, et surtout les cheveux.

On sait que la technique la plus usuelle pour obtenir une déformation permanente des cheveux consiste, dans un premier temps, à réaliser l'ouverture des liaisons disulfures - S-S- de la kératine (cystine) à l'aide d'une composition réductrice, contenant un agent réducteur (étape de réduction) puis, après avoir de préférence, rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de la première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème des techniques de permanentes connues à ce jour est que leur application répétée sur les cheveux induit à la longue une altération progressive de la qualité du cheveu, et notamment une altération progressive et marquée de la brillance et des propriétés cosmétiques du cheveu, en particulier au niveau de la douceur des fibres qui ont tendance à devenir de plus en plus rêches ainsi qu'au niveau de leur démêlage, les cheveux devenant de plus en plus difficiles à démêler. Cette altération est en outre particulièrement accentuée lorsque l'étape de fixation de l'opération de déformation permanente est effectuée à l'aide d'un bromate.

Pour limiter cette altération des cheveux, il a déjà été proposé d'introduire directement, dans la composition réductrice, des agents de conditionnement. Par exemple, les demandes de brevet japonais H2-250814 et H9-151120 décrivent des compositions réductrices contenant des silicones aminées, pouvant éventuellement se présenter sous la forme de microémulsion.

Cependant, les procédés de déformation permanente des cheveux utilisant de telles compositions ne donnent pas encore entière satisfaction, dans la mesure où le degré, la qualité et la nervosité de frisure sont généralement insuffisants et éphémères, comme si l'agent de conditionnement, notamment les silicones aminées, directement associé à l'agent réducteur, freinaient l'activité de ce dernier.

Le problème posé par l'invention est de fournir un procédé de déformation permanente des fibres kératiniques, notamment des cheveux, qui réduise la dégradation mécanique et/ou cosmétique des cheveux, tout en apportant un degré, une qualité et une nervosité de frisure satisfaisants.

La Demanderesse a découvert, de manière surprenante et inattendue, qu'en appliquant sur les cheveux, avant d'appliquer la composition réductrice et/ou après avoir appliqué la composition oxydante, au moins une composition cosmétique de pré-traitement et/ou de post-traitement contenant au moins une silicone aminée bien particulière, il était possible de résoudre le problème posé par la présente invention.

L'invention a pour objet un procédé de déformation permanente des fibres kératiniques, notamment des cheveux, comprenant au moins les opérations consistant à :
(i) appliquer sur les fibres kératiniques une composition réductrice ;
(ii)réaliser l'oxydation des fibres kératiniques,
caractérisé par le fait qu'il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, au moins une silicone aminée comprenant au moins un groupement aminoéthyliminoalkyl(C₄-C₈).

Un autre objet de l'invention concerne un kit pour la déformation permanente des fibres kératiniques, l'un des compartiments contenant une composition cosmétique de pré-traitement et/ou de post-traitement comprenant au moins une silicone aminée comprenant au moins un groupement aminoéthyliminoalkyl(C₄-C₈).

Les silicones aminées selon l'invention présentent, avantageusement, la formule suivante : 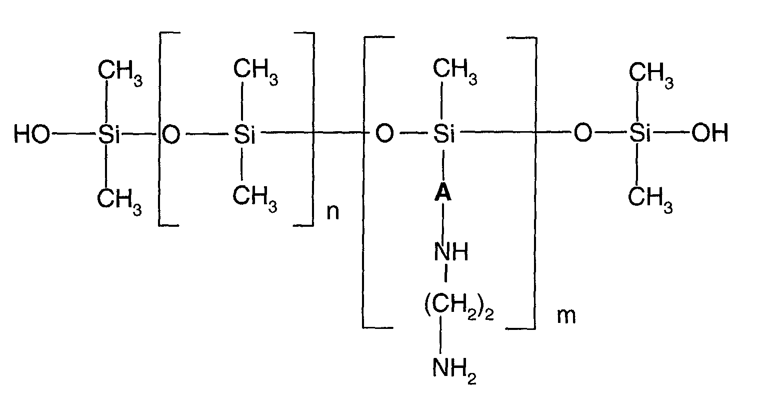 dans laquelle :
A désigne un radical alkylène linéaire ou ramifié en C₄-C₈ et de préférence en C₄
m et n sont des nombres tels que la somme (n + m) peut varier notamment de 1 à 2 000 et en particulier de 50 à 150, n pouvant désigner un nombre allant de 0 à 1 999 et notamment de 49 à 149 et m pouvant désigner un nombre allant de 1 à 2 000, et notamment de 1 à 10.

La viscosité de la silicone est de préférence supérieure à 25 000 mm²/s à 25°C.

Plus préférentiellement, cette viscosité peut aller de 30 000 à 200 000 mm²/s à 25°C et encore plus préférentiellement de 30 000 à 150 000 mm²/s à 25°C.

Conformément à l'invention, la viscosité est mesurée par exemple selon la norme ASTM 445 Appendice C.

La silicone aminée présente une masse moléculaire moyenne en poids allant, de préférence, de 2000 à 1000000 et encore plus particulièrement de 3500 à 200000.

Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.
Ces silicones peuvent être mises en oeuvre tel quel ou sous forme d'émulsions. L'émulsion peut comprendre un ou plusieurs tensioactifs. Les tensioactifs peuvent être de toute nature mais de préférence cationique et/ou non ionique.
Les particules de silicone dans l'émulsion ont une taille moyenne de 3 nm à 500 nm, de préférence de 70 nm à 500 nm et encore plus préférentiellement de 150 à 275 nm.

Une silicone répondant à cette formule est par exemple la DC2-8299® de la société DOW CORNING.

Avantageusement, la concentration en silicones aminées comprenant au moins un groupement aminoéthyliminoalkyl(C₄-C₈)dans la composition de pré-traitement ou de post-traitement est comprise entre 0,05 et 10 % en poids par rapport au poids total de cette composition, et plus avantageusement entre 0,1,et 7 %.

Les compositions de pré-traitement ou de post-traitement contenant au moins une silicone aminée telle que définie ci-dessus peuvent contenir des actifs hydrosolubles ou liposolubles, ayant une activité cosmétique ou dermopharmaceutique. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes, des solvants et des parfums.

Elles peuvent aussi comprendre des solvants et en particulier des alcools inférieurs en C1C8 tels que l'éthanol.

De préférence, le pH de la composition de pré-traitement ou de post-traitement comprenant au moins une silicone aminée telle que définie ci-dessus est compris de préférence entre 2 et 10, et plus préférentiellement entre 3 et 9.

La composition de pré-traitement comprenant au moins une silicone aminée telle que définie ci-dessus est appliquée sur les cheveux à traiter, lesquels auront été, éventuellement, préalablement mouillés. Cette application peut être réalisée après l'habituelle étape de mise sous tension des cheveux sous une forme correspondant à la forme finale désirée pour ces derniers (boucles par exemple), cette étape pouvant, elle-même, être mise en oeuvre par tout moyen, mécanique notamment, approprié et connu en soi pour maintenir sous tension des cheveux, tels que par exemple rouleaux, bigoudis et analogues.

Avantageusement, on laisse agir, à température ambiante ou sous chaleur, sur les cheveux la composition de pré-traitement ou de post-traitement comprenant au moins une silicone aminée telle que dédinie ci-dessus pendant une durée comprise entre 1 et 60 minutes, et plus avantageusement entre 3 et 30 minutes.

Selon une étape facultative du procédé, on peut rincer les cheveux imprégnés de la composition de pré-traitement comprenant au moins une silicone aminée telle que définie ci-dessus, le rinçage étant effectué généralement à l'eau.

Dans une étape indispensable du procédé selon l'invention, on applique sur les cheveux une composition réductrice, ladite composition réductrice contenant généralement au moins un thiol.

Le thiol de la composition réductrice peut être choisi parmi les thiols connus comme agents réducteurs tels que par exemple l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide 3-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)ω-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

On utilise de préférence l'acide thioglycolique, l'acide thiolactique et l'acide 3-mercaptopropionique.

Les agents réducteurs sont généralement présents à une concentration qui peut être comprise entre 1 et 20 % en poids par rapport au poids total de la compositon réductrice.

Le pH de la composition réductrice est, généralement, compris entre 6 et 10 et de préférence entre 7 et 9.

Les pH des compositions réductrices peuvent être ajustés clasiquement par ajout d'agents basifiants, tels que par exemple l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires ou un carbonate organique tel que le carbonate de guanidine, tous ces composés pouvant bien entendu être pris seuls ou en mélange.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur les cheveux. Cette phase d'attente est effectuée généralement en laissant reposer à l'air libre (à température ambiante ou avec chauffage) la chevelure traitée. Pendant cette phase d'attente, on prend généralement soin que les cheveux ne sèchent pas complètement et restent humides.

Les cheveux imprégnés de la composition réductrice peuvent être, ensuite, rincés soigneusement, généralement à l'eau. Eventuellement, après rinçage, on met en oeuvre une phase de chauffage à température élevée pendant quelques secondes.

Puis, on applique sur les cheveux ainsi rincés une composition oxydante dans le but de fixer la nouvelle forme imposée aux cheveux. Il peut également être envisagé de laisser les cheveux s'oxyder à l'air.

La composition oxydante contient un agent oxydant qui peut être choisi parmi l'eau oxygénée, un bromate alcalin, un persel ou un polythionate. Comme indiqué précédemment, l'un des grands avantages du procédé selon l'invention est qu'il convient parfaitement dans le cas des compositions oxydantes à base de bromates. La concentration en bromates dans la composition oxydante est généralement comprise entre 0,1 et 2 M.

Le pH de la composition oxydante est généralement compris entre 2 et 10.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

La composition de post-traitement comprenant au moins une silicone aminée telle que définie ci-dessus est, de préférence, appliquée après rinçage de la composition oxydante, sur des cheveux humides ou secs. Les cheveux ayant subi le post-traitement peuvent éventuellement être séchés et/ou chauffées et/ou rincés, avant le coiffage. Le cas échéant, la composition peut être appliquée alors que les cheveux sont maintenus par un dispositif mécanique, comme des rouleaux de mise en pli ou des bigoudis.

Le plus souvent, les cheveux imprégnés de la composition oxydante sont rincés soigneusement, généralement à l'eau. On sépare, avant ou après le rinçage, la fibre kératinique des moyens nécessaire à la mise sous tension.

On obtient finalement une chevelure présentant de bonnes propriétés cosmétiques : les cheveux sont plus brillants, plus doux et plus faciles à démêler ou à coiffer.

De manière avantageuse, la composition de pré-traitement ou de post-traitement contenant au moins une silicone aminée telle que définie ci-dessus est appliquée selon l'une au moins des variantes suivantes :
- sur des cheveux propres et humides, avant la mise en oeuvre des moyens de mise sous tension des cheveux, sans rincer les cheveux avant l'application du réducteur ;
- sur des cheveux humides après rinçage du fixateur, les cheveux étant ultérieurement soit rincés, soit séchés.

Lorsque le procédé de déformation permanente des cheveux est un procédé de défrisage, on peut, de manière connue en soi, mettre en oeuvre, un défrisant généralement soit thiolé, soit alcalin.

Dans le cas de défrisant thiolé, le procédé conforme à l'invention est avantageusement mis en oeuvre en appliquant la composition de pré-traitement ou de post-traitement contenant au moins une silicone aminée telle que définie ci-dessus selon l'une au moins des variantes suivantes :
- sur des cheveux propres et humides, sans rinçage avant l'application du réducteur ;
- sur des cheveux propres et humides, après le rinçage du fixateur, en rinçant avant le séchage des cheveux.

Dans le cas de défrisant alcalin, le procédé conforme à l'invention est avantageusement mis en oeuvre en appliquant la composition de pré-traitement ou de post-traitement contenant au moins une silicone aminée telle que définie ci-dessus sur cheveux humides, après rinçage de shampooing neutralisant, en rinçant avant le séchage des cheveux.

Dans le cas d'un frisage des cheveux, le procédé conforme à l'invention procure des boucles nerveuses et les cheveux sont spécialement souples, légers, soyeux et bien individualisés.

Dans le cas d'un défrisage des cheveux, le procédé conforme à l'invention procure notamment une maîtrise du volume de la chevelure, un lissage des cheveux de la racine à la pointe et un toucher plus naturel.

L'invention pourra être mieux comprise à l'aide des exemples non limitatifs qui suivent et qui constituent des modes de mise en oeuvre avantageux du procédé selon l'invention.

m.a. signifie matière active.

### EXEMPLES :

*1) Composition de soin protecteur avant permanente*

| Ingrédients | % ma |
|---|---|
| - alcool cétylstéarylique / laurylsulfate de sodium / myristate de cétyle / alcool myristique (62/20/8/10) | 12 |
| - alcool oléique oxyéthyléné (20 OE) | 0.1 |
| - glycérine | 0.5 |
| - PDMS à groupements aminoéthyl aminobutyl α,ω-disilanol en émulsion cationique (DC2-8299 de Dow Corning) | 2 |
| - eau déminéralisée | qsp 100 g |

*2) Composition de soin des cheveux défrisés et permanentes*
La composition décrite en 1) peut s'utiliser en soin sur cheveux récemment défrisés ou permanentés. Cette composition est appliquée sur cheveux humides après le rinçage du shampooing neutralisant, ou après le rinçage du fixateur dans le cas d'une permanente. Cette composition apporte lissage et douceur à la chevelure.

## Revendications

1. Procédé de déformation permanente des fibres kératiniques, notamment des cheveux, comprenant au moins les opérations consistant à :
(i) appliquer sur les fibres kératiniques une composition réductrice ;
(ii) réaliser l'oxydation des fibres kératiniques,
**caractérisé par le fait qu'**il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, au moins une silicone aminée comprenant au moins un groupement aminoéthyliminoalkyl(C₄-C₈).

2. Procédé selon la revendication 1, **caractérisé par le fait que** la silicone aminée répond à la formule : 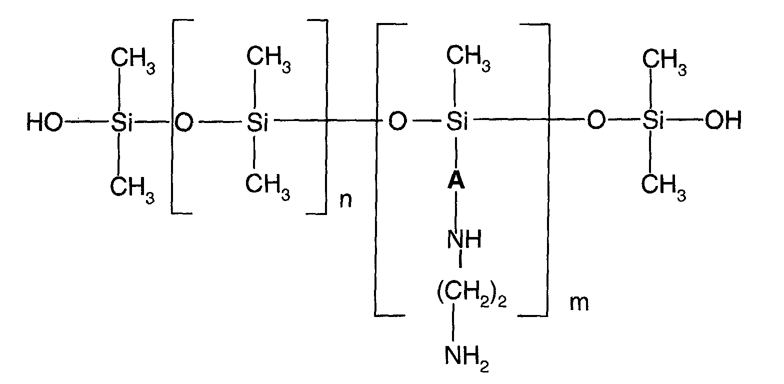 dans laquelle :
A désigne un radical alkylène linéaire ou ramifié en C₄-C₈ et de préférence en C₄
m et n sont des nombres tels que la somme (n + m) peut varier notamment de 1 à 2 000 et en particulier de 50 à 150, n pouvant désigner un nombre allant de 0 à 1 999 et notamment de 49 à 149 et m pouvant désigner un nombre allant de 1 à 2 000, et notamment de 1 à 10.

3. Procédé selon l'une quelconque des revendications précédentes, **caractérisé par le fait que** la viscosité de la silicone est supérieure à 25 000 mm²/s à 25°C.

4. Procédé selon l'une quelconque des revendications précédentes, **caractérisé par le fait que** la viscosité de la silicone aminée peut aller de 30 000 à 200 000 mm²/s à 25°C, et encore plus préférentiellement de 30 000 à 150 000 mm²/s à 25°C.

5. Procédé selon l'une quelconque des revendications précédentes, **caractérisé par le fait que** la silicone aminée la silicone aminée présente une masse moléculaire moyenne en poids allant de 2000 à 1000000, et encore plus particulièrement de 3500 à 200000.

6. Procédé selon l'une quelconque des revendications précédentes, **caractérisé par le fait que** le pH de la composition de pré-traitement ou de post-traitement est compris de préférence entre 2 et 10, et plus préférentiellement entre 3 et 9.

7. Procédé selon l'une quelconque des revendications précédentes, **caractérisé par le fait que** la concentration en silicones aminées comprenant au moins un groupement aminoéthyliminoalkyl(C₄-C₈) dans la composition de pré-traitement ou de post-traitement est comprise entre 0,05 et 10 % en poids par rapport au poids total de cette composition, et plus avantageusement entre 0,1,et 7 %.

8. Procédé selon l'une quelconque des revendications précédentes, **caractérisé par le fait qu'**on laisse agir sur les cheveux la composition de pré-traitement ou de post-traitement pendant une durée comprise entre 1 et 60 minutes, et plus avantageusement entre 3 et 30 min.

9. Procédé selon l'une quelconque des revendications précédentes, **caractérisé par le fait que** la composition de pré-traitement ou de post-traitement comprend, en outre, des additifs choisis parmi les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes , des solvants et des parfums.

10. Kit pour la déformation permanente des fibres kératiniques, l'un des compartiments contenant une composition cosmétique de pré-traitement et/ou de post-traitement comprenant au moins une silicone aminée telle que définie dans l'une quelconque des revendications 1 à 7.

11. Utilisation d'une composition cosmétique de pré-traitement et/ou de post-traitement comprenant, dans un véhicule cosmétiquement acceptable, au moins une silicone aminée telle que définie dans l'une quelconque des revendications 1 à 7 pour la déformation permanente des fibres kératiniques.
